(19) Europäisches Patentamt
European Patent Office
Office européen des brevets

(11) Numéro de publication: **0 181 826**
**A1**

(12) **DEMANDE DE BREVET EUROPEEN**

(21) Numéro de dépôt: 85420193.6

(22) Date de dépôt: 24.10.85

(51) Int. Cl.[4]: C 07 D 235/24
A 61 K 31/415

(30) Priorité: 26.10.84 FR 8416620

(43) Date de publication de la demande: 21.05.86 Bulletin 86/21

(84) Etats contractants désignés: AT BE CH DE FR GB IT LI LU NL SE

(71) Demandeur: RHONE-POULENC AGROCHIMIE
14-20, rue Pierre Baizet
F-69009 Lyon(FR)

(72) Inventeur: Giraudon, Raymond
5 rue des Colverts
F-77330 - Lesigny(FR)

(72) Inventeur: Santini, Georges
92, rue Bugeaud
F-69006 Lyon(FR)

(74) Mandataire: Brachotte, Charles et al,
RHONE-POULENC AGROCHIMIE P.I.D. BP 9163
F-69263 Lyon Cedex 09(FR)

(54) **Nouveaux dérivés du cyano-2 benzimidazole, leur préparation et leur utilisation comme fongicides et acaricides.**

(57) L'invention concerne de nouveaux dérivés du cyano-2 benzimidazole.

Ces composés répondent à la formule (II):

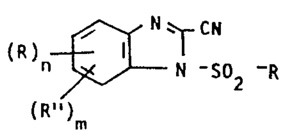

(II)

avec :

n : 0, 1 ou 2,

m : 1 ou 2,

R : halogène ; alkyle ou alkoxy ou alkylthio inférieurs éventuellement halogéné ; alkoxy inférieur éventuellement substitué par un ou plusieurs halogènes ; $NO_2$–, CN–,

R' : alkyle ou cycloalkyle inférieurs éventuellement halogènés, amino éventuellement substitués par un ou deux radicaux alkyles, ou un atome d'azote substitué par deux radicaux formant avec cet atome d'azote un hétérocycle comportant de 4 à 6 chaînons et de la à 3 hétéroatomes dans le cycle.

Ils sont utilisables en agriculture notamment pour la lutte contre les champignons phytopathogènes.

R" : alcénylthio inférieur, alcényloxy inférieur, alcynylthio inférieur, alcynyloxy inférieur ou aralkylthio éventuellement substitués.

EP 0 181 826 A1

La présente demande de brevet concerne de nouveaux dérivés du cyano-2 benzimidazole ainsi que la préparation de ces composés. Elle concerne de plus les compositions pesticides, notamment antifongiques utilisables en agriculture et contenant comme matière active un de ces composés ainsi que les traitements pesticides et plus particulièrement les traitements antifongiques effectués au moyen de ces composés.

Le brevet américain n° 3 576 818 décrit des dérivés du cyano-2 benzimidazole répondant à la formule (I)

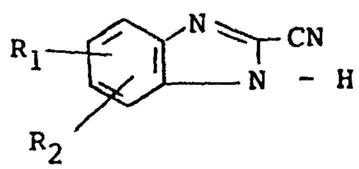

(I)

dans laquelle $R_1$ et $R_2$ sont choisis dans le groupe comprenant l'hydrogène, le chlore, le brome, $NO_2$ et les radicaux alkyle et alkoxy contenant au plus 4 atomes de carbone. Ce brevet américain indique que ces composés (I) sont utilisables comme bactéricides et agents antiseptiques, il décrit dans son exemple n° 1 le cyano-2 benzimidazole, c'est à dire le composé selon la formule (I) pour lequel $R_1$ et $R_2$ représentent l'atome d'hydrogène.

La demande de brevet français n° 2 051 493 décrit des dérivés du cyano-2 benzimidazole pour lesquels l'atome d'azote en position 1 sur le cycle benzimidazole peut-être substitué par un atome d'hydrogène ou un radical alkyle ou alkoxycarbonyle. Elle signale que ces composés ont une activité biocide et sont utilisables comme fongicides, anthelminthiques, insecticides et herbicides. L'exemple n° 4 de cette demande de brevet français décrit le cyano-2 benzimidazole, déjà mentionné ci-dessus.

La demande de brevet français n° 2 070 090 décrit des dérivés du cyano-2 benzimidazole pour lesquels l'atome

d'azote en position 1 sur le cycle benzimidazole peut-être substitué par l'atome d'hydrogène ou un radical alkyle, alkoxyalkyle, alcényle, carboxyalkyle, alcanoyle, aminoalkyle, hydroxy, alkoxy, carboxyalkoxy, sulfoalkoxy et aminoalkoxy et enseigne que ces composés sont utilisables comme agents anthelminthiques, comme agents anti-coccidies et comme pesticides par exemple herbicides et bactéricides.

La présente demande de brevet concerne des dérivés du cyano-2 benzimidazole différents de ceux décrits dans les documents cités plus haut et présentant de remarquables propriétés antifongiques.

Les nouveaux dérivés du cyano-2 benzimidazole selon l'invention sont caractérisés en ce qu'ils répondent à la formule générale : (II)

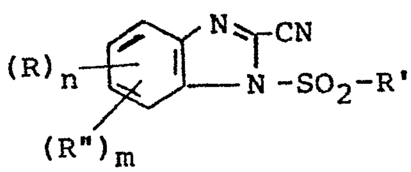

(II)

dans laquelle n, R et R' ont les significations indiquées ci-après, étant entendu que, dans ce qui suit, sauf indication contraire, l'adjectif "inférieur" appliqué à un radical organique signifie que ce radical comporte au plus six atomes de carbone.

Dans la formule (II) :

n représente un nombre entier pouvant être égal à 0, 1 ou 2

m représente un nombre entier égal à 1 ou 2

R représente un atome d'halogène ou un radical alkyle inférieur éventuellement substitué par un ou plusieurs atomes d'halogène ; alkoxy inférieur éventuellement substitué par un ou plusieurs atomes d'halogène ; alkylthio inférieur éventuellement substitué par un ou plusieurs atomes d'halogène ; nitro ; cyano, étant

entendu que, lorsque n est égal à 2, les substituants R peuvent être soit identiques, soit différents,

R' représente un radical alkyle ou cycloalkyle inférieurs éventuellement substitués par un ou plusieurs atomes d'halogène (tel que, par exemple, les radicaux méthyle, éthyle, isopropyle, trichlorométhyle, trifluorométhyle, etc...) ; ou un radical amino, éventuellement substitué par un ou deux radicaux alkyles inférieurs, identiques ou différents, eux-mêmes éventuellement substitués (par exemple par un ou plusieurs halogènes ou alkoxy ou alkylthio inférieurs) ; ou un atome d'azote substitué par deux radicaux formant avec cet atome d'azote un hétérocycle, lui-même éventuellement substitué, comportant de 4 à 6 chainons et de 1 à 3 hétéroatomes dans le cycle (tel que par exemple le radical morpholino, le radical pyrrolidino etc...).

R" représente un radical alcénylthio inférieur, alcényloxy inférieur, alcynylthio inférieur, alcynyloxy inférieur, aralkylthio, lesdits radicaux étant eux-mêmes éventuellement substitués, étant entendu que lorsque m est égal à 2, les substituants R"peuvent être soit identiques, soit différents.

De préférence :

n est égal à 0 ou à 1,

R représente un atome d'halogène ou le radical cyano, nitro, trifluorométhyle, trifluorométhoxy ou trifluorométhylthio,

R' représente le radical diméthylamino ou un radical alkyle ($C_1$-$C_3$) éventuellement halogéné,

m est égal à 1,

R" représente le radical propargylthio ou un radical benzylthio.

Au sens du présent texte, on entendra que les radicaux ou parties alkyles peuvent être soit linéaires, soit ramifiés. Il en est de même des radicaux alcényles et

alcynyles.

L'invention concerne de plus un procédé pour préparer les composés selon la formule (II).

Ce procédé est caractérisé en ce qu'il consiste à faire réagir un cyano-2 benzimidazole de formule (III)

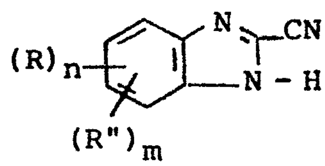

(III)

dans laquelle R, R", m et n ont le même signification que dans la formule (II), ou un sel de métal alcalin ou d'ammonium de ce cyano-2 benzimidazole (III), sur un halogénure de formule (IV)

$$X\text{-}SO_2R' \qquad (IV)$$

dans laquelle R' a la même signification que dans la formule (II) et X représente un atome d'halogène, de préférence un atome de chlore.

La réaction du cyano-2 benzimidazole (III) sur l'halogènure (IV) s'effectue avantageusement en présence d'un accepteur d'acide, en milieu anhydre ou non anhydre, dans un solvant, inerte dans les conditions de la réaction, généralement à la température d'ébullition du solvant. Comme accepteurs d'acide, on peut mentionner des bases minérales comme par exemple la soude ou la potasse, les carbonates de métaux alcalins ou de métaux alcalinoterreux, des bases azotées comme la triéthylamine. Comme solvants on utilise avantageusement des solvants aprotiques polaires, tels que par exemple, le diméthylformamide, le diméthylacétamide, le diméthylsulfoxyde, l'acétone, la méthyléthylcétone, l'acétonitrile, la N-méthylpyrrolidone. Si désiré, cette réaction peut être effectuée en présence d'un catalyseur approprié. Comme catalyseur

utilisable, on peut mentionner des catalyseurs de transfert de phases, tels que, par exemple, des dérivés d'ammonium quaternaire.

La réaction du sel de métal alcalin ou d'ammonium du cyano-2 benzimidazole (III) sur l'halogénure (IV) ne nécessite pas la présence d'un accepteur d'acide. Elle s'effectue en milieu anhydre ou non anhydre, dans un solvant, inerte dans les conditions de la réaction, généralement à la température d'ébullition du solvant. Les solvants aprotiques polaires cités plus haut peuvent être utilisés avantageusement dans cette réaction.

Si désiré, cette réaction peut être effectuée en présence d'un catalyseur approprié, tel que, par exemple, un catalyseur de transfert de phases (par exemple un dérivé d'ammonium quaternaire).

Le sel de métal alcalin ou d'ammonium du composé (III) est préparé dans une opération préalable, éventuellement effectuée in situ, par action d'une base appropriée (par exemple soude, potasse, ammoniaque) ou d'un carbonate de métal alcalin, ou d'un alcoolate de métal alcalin (par exemple méthylate de sodium ou éthylate de sodium ou potassium) sur ce composé (III).

En fin de réaction, quelque soit le procédé utilisé, le composé formé est isolé du milieu réactionnel par tout moyen en soi connu tel que, par exemple par distillation du solvant, ou par cristallisation du produit dans le milieu réactionnel, ou par filtration, puis, si nécessaire, ce composé est purifié par des méthodes usuelles telles que recristallisation dans un solvant approprié.

Le cyano-2 benzimidazole de formule (III), servant de produit de départ peut être préparé selon l'une ou l'autre des méthodes A, B et C décrites ci-après :

<u>Méthode A</u> :

Action de l'ammoniaque sur un trihalogénométhyl-2

benzimidazole, en opérant selon le procédé décrit dans le brevet américain n° 3 576 818 cité plus haut,

<u>Méthode B</u> :

Action du trichlorure de phosphore sur un cyano-2 hydroxy-1 benzimidazole de formule (V)

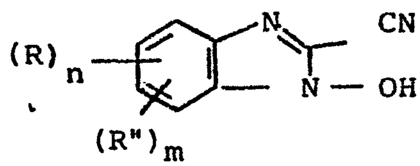

(V)

dans laquelle R, R", m et n ont la même signification que dans la formule (II), en opérant dans l'acétone chauffée à la température du reflux.

Le cyano-2 hydroxy-1 benzimidazole (V) peut être obtenu à partir du (nitro-2 phényl) amino-2 acétonitrile de formule (VI)

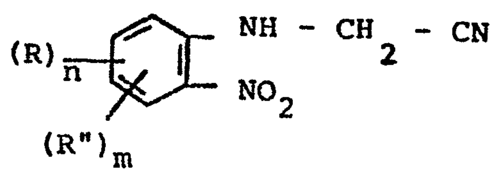

(VI)

dans laquelle R, R", m et n ont la même signification que précédemment, par chauffage en présence de carbonate de potassium, selon la méthode décrite par L. KONOPSKI et B. SERAFIN dans Rocz. Chem. <u>51</u> 1783 (1977).

Le composé (VI) peut être obtenu à partir de la nitro-2 aniline correspondante en opérant selon la méthode décrite par K. DIMROTH et H.C. AURICH dans Chem. Ber. <u>98</u> 3902 (1965).

<u>Méthode C</u> :

Action d'un agent de déshydratation tel que $POCl_3$

ou $SOCl_2$ sur l'hydroxyiminométhyl-2 benzimidazole de formule (VII)

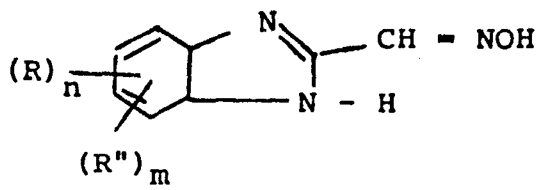

(VII)

dans laquelle R, R", m et n ont la même signification que dans la formule (II)

Les composés répondant aux formules (III), (V) et (VI) sont compris dans le cadre de la présente invention, en tant que moyens spécialement conçus pour la mise en oeuvre du procédé de préparation décrit plus haut.

Les exemples ci-après, décrits à titre non limitatif, illustrent la préparation des composés selon l'invention et leur utilisation comme fongicides. Les structures des composés décrits dans ces exemples ont été confirmées par spectrométrie de résonance magnétique nucléaire (RMN) et/ou par spectrométrie infrarouge.

Exemple 1

Préparation du cyano-2 diméthylsulfamoyl-1 (propyne-2 yl thio)-5 benzimidazole (composé 1A) en mélange avec le cyano-2 diméthylsulfamoyl-1 (propyne-2 yl thio)-6 benzimidazole (composé 1B).

A une suspension de 6,2 g (0,029 mole) de cyano-2 (propyne-2 yl thio)-5 benzimidazole dans 60 ml d'acétone séchée sur $K_2CO_3$, on ajoute 1,85 g de potasse en écailles.

Après 30 minutes environ, la potasse est dissoute et le mélange réactionnel est devenu presque homogène.

On ajoute alors en une fois 3,12 ml (0,029 mole) de

chlorure de diméthylsulfamoyle et le mélange réactionnel est agité pendant 12 heures à température ordinaire. Il blanchit et s'échauffe légèrement.

Le mélange réactionnel est ensuite concentré sous vide partiel, puis repris par 2 x 100 ml de $CH_2Cl_2$, filtré et chromatographié sur 200 g de silice avec $CH_2Cl_2$.

On obtient un solide jaune huileux que l'on recristallise dans 200 ml de pentane. On obtient ainsi 5,2 g (Rendement 56 %) d'un solide jaunâtre fondant à 87°C. Par spectrographie RMN et infra-rouge, ce composé est analysé comme étant un mélange en proportions sensiblement égales de cyano-2 diméthylsulfamoyl-1 (propyne-2 yl thio)-5 benzimidazole (composé 1A) et de cyano-2 diméthylsulfamoyl-1 (propyne-2 yl thio)-6 benzimidazole (composé 1B).

Exemple 2

En opérant selon la méthode décrite dans l'exemple précédent, le mélange ci-après a été préparé :

- Cyano-2 benzylthio-5 diméthylsulfamoyl-1 benzimidazole (composé 2A) et cyano-2 benzylthio-6 diméthylsulfamoyl-1 benzimidazole (composé 2B), sous la forme d'un mélange 50/50, fondant à 96°C.

Exemple 3

Test en serre sur mildiou de la tomate

Des plants de tomate (Lycopersicum esculentum), de variété Marmande, sont cultivés dans des godets. Lorsque ces plants sont agés d'un mois (stade 5 à 6 feuilles, hauteur 12 à 15 cm), ils sont traités par pulvérisation au moyen d'une suspension ou solution aqueuse de la matière à tester, à la concentration désirée et contenant 0,02% d'un condensat de monoléate de sorbitan et de 20 molécules d'oxyde d'éthylène. Chaque plant de tomate reçoit environ 5ml de la solution ou dispersion. Pour chaque concentration de matière active à tester, le traitement est effectué sur huit plants. Des plants utilisés comme témoins sont traités

par une solution ne contenant pas de matière active, mais contenant 0,02% du même condensat de monooléate de sorbitan et d'oxyde d'éthylène.

Après séchage pendant 4 heures, on contamine chaque plant par pulvérisation au moyen d'une suspension aqueuse de spores de Phytophthora infestans, responsable du mildiou de la tomate, à raison d'environ 1 ml/plant (soit environ $2.10^5$ spores par plant).

Après cette contamination, les plants de tomate sont mis en incubation pendant deux à trois jours à 17°C environ en atmosphère saturée d'humidité, puis pendant quatre jours à 22°C environ sous 70% à 80% d'humidité relative.

Sept jours après la contamination, on compare les résultats obtenus dans le cas des plants traités par la matière active à tester à ceux obtenus dans le cas des plants utilisés comme témoins et on détermine la concentration minimale inhibitrice entraînant de 95 à 100% d'inhibition du développement du champignon considéré (CMI 95-100).

Dans ces conditions, on observe que, pour les composés ou mélanges de composés décrits dans les exemples précédents, cette concentration a été respectivement la suivante :

| Composé ou mélange testé | CMI (95-100) Phytophthora infestans en mg/l |
|---|---|
| 1A + 1B | inférieur à 125 |

Exemple 4

Test en serre sur mildiou du tabac

On opère comme à l'exemple précédent si ce n'est que les végétaux sont des plants de tabac (Nicotiana tabacum) de variété Samson et que ces plants sont contaminés par des spores de Peronospora tabacina, responsable du

mildiou du tabac.

Dans ces conditions, on a observé que, pour les composés ou mélanges de composés décrits dans les exemples précédents, les concentrations minimales inhibitrices entraînant de 95 à 100% d'inhibition du champignon considéré (CMI 95-100) sont respectivement les suivantes :

| Composés ou mélange testés | CMI (95-100) Peronospora tabacina en mg/l |
|---|---|
| 1A + 1B | ...........inférieur à 500 |

Les composés selon l'invention sont avantageusement utilisés comme antifongiques dans le domaine agricole. Ils présentent une action par contact et par systèmie et peuvent être employés à tire préventif et/ou à titre curatif pour la lutte contre divers champignons phytopathogènes tels que par exemple de nombreux phycomycètes et basidiomycètes. Certains de ces composés peuvent être également utilisés avantageusement comme antiacariens notamment vis à vis des acariens phytophages.

Pour leur emploi dans la pratique, les composés selon l'invention ne sont généralement pas utilisés seuls. Le plus souvent ils sont utilisés dans des compositions qui comprennent en général, en plus de la matière active, un support (ou diluant) inerte et/ou un agent tensioactif compatibles avec la matière active.

Ces compositions font également partie de la présente invention. Elles contiennent habituellement de 0,001 à 95% en poids de matière active. Leur teneur en agent tensioactif est en général comprise entre 0% et 20% en poids.

Par le terme "support", dans le présent exposé, on désigne une matière organique ou minérale, naturelle ou

synthétique, avec laquelle la matière active est associée pour faciliter son application sur la plante, sur des graines ou sur le sol. Ce support est donc généralement inerte et il doit être acceptable en agriculture, notamment par la plante traitée. Le support peut être solide (argiles, silicates naturels ou synthétiques, silice, craies, résines, cires, engrais solides, etc...) ou liquide (eau, alcools, cétones, fractions de pétrole, hydrocarbures aromatiques ou paraffiniques, hydrocarbures chlorés, gaz liquéfiés, etc...).

L'agent tensioactif peut être un agent émulsionnant, dispersant ou mouillant de type ionique ou non ionique. On peut citer par exemple des sels d'acides polyacryliques ; des sels d'acides lignosulfoniques;des sels d'acides phénolsulfoniques ou naphtalènesulfoniques ; des polycondensats d'oxyde d'éthylène sur des alcools gras ou sur des acides gras ou sur des amines grasses ou sur des phénols substitués (notamment des alkylphénols ou des arylphénols ou le styrylphénol) ; des sels d'esters d'acides sulfosucciniques ; des dérivés de la taurine (notamment des alkyltaurates) ; des esters phosphoriques d'alcools ou de phénols polyoxyéthylés. La présence d'au moins un agent tensioactif est généralement indispensable surtout lorsque le support inerte n'est pas soluble dans l'eau, et que l'agent vecteur de l'application est l'eau.

Les compositions utilisées dans l'invention peuvent être sous des formes assez diverses, solides ou liquides.

Comme formes de compositions solides, on peut citer les poudres pour poudrage (à teneur en matières actives pouvant aller jusqu'à 100 %).

Comme formes de compositions liquides ou destinées constituer des compositions liquides lors de l'application, on peut citer les solutions, en particulier les concentrés solubles dans l'eau, les concentrés émulsionnables, les émulsions, les suspensions concentrées, les aérosols, les

poudres mouillables (ou poudre à pulvériser), les pâtes.

Les concentrés émulsionnables ou solubles comprennent le plus souvent 10 à 80 % de matière active, les émulsions ou solutions prêtes à l'application contenant, quant à elles, 0,001 à 20 % de matière active. En plus du solvant, les concentrés émulsionnables peuvent contenir, quand c'est nécessaire, un co-solvant approprié et de 2 à 20 % d'additifs appropriés, comme des stabilisants, des agents tensioactifs, notamment des émulsifs, des agents de pénétration, des inhibiteurs de corrosion, des colorants, des adhésifs.

A partir de ces concentrés, on peut obtenir par dilution avec de l'eau des émulsions de toute concentration désirée, qui conviennent particulièrement à l'application sur les cultures.

Les suspensions concentrées, également applicables en pulvérisation, sont préparées de manière à obtenir un produit fluide stable ne se déposant pas et elles contiennent habituellement de 10 à 75 % de matières actives, de 0,5 à 15 % d'agents tensioactifs, de 0,1 à 10 % d'agents thixotropes, de 0 à 10 % d'additifs appropriés, comme des anti-mousses, des inhibiteurs de corrosion, des stabilisants, des agents de pénétration et des adhésifs et, comme support, de l'eau ou un liquide organique dans lequel la matière active est peu ou pas soluble : certaines matières solides organiques ou des sels minéraux peuvent être dissous dans le support pour aider à empêcher la sédimentation ou comme antigels pour l'eau.

Les poudres mouillables (ou poudre à pulvériser) sont habituellement préparées de manière qu'elles contiennent 20 à 95 % de matières actives, et elles contiennent habituellement, en plus du support solide, de 0 à 5 % d'un agent mouillant, de 3 à 10 % d'un agent dispersant, et, quand c'est nécessaire, de 0 à 10 % d'un ou plusieurs stabilisants et/ou autres additifs, comme des agents de

pénétration, des adhésifs, ou des agents antimottants, colorants, etc...

Pour obtenir ces poudres à pulvériser ou poudres mouillables, on mélange intimement la matière active dans des mélangeurs appropriés avec les substances additionnelles et on broie avec des moulins ou autres broyeurs appropriés. On obtient par là des poudres à pulvériser dont la mouillabilité et la mise en suspension sont avantageuses ; on peut les mettre en suspension avec de l'eau à toute concentration désirée et cette suspension est utilisable très avantageusement en particulier pour l'application sur les feuilles des végétaux.

Comme cela a déjà été dit, les dispersions et émulsions aqueuses, par exemples des compositions obtenues en diluant à l'aide d'eau une poudre mouillable ou un concentré émulsionnable selon l'invention, sont comprises dans le cadre général des compositions utilisables dans la présente invention. Les émulsions peuvent être du type eau-dans-l'huile ou huile-dans-l'eau et elles peuvent avoir une consistance épaisse comme celle d'une "mayonnaise".

Les granulés destinés à être disposés sur le sol sont habituellement préparés de manière qu'ils aient des dimensions comprises entre 0,1 et 2 mm et ils peuvent être fabriqués par agglomération ou imprégnation. En général, les granulés contiennent 0,5 à 25 % de matière active et 0 à 10 % d'additifs comme des stabilisants, des agents de modification à libération lente, des liants et des solvants.

Les composés de formule (II) peuvent encore être utilisés sous forme de poudres pour poudrage, on peut ainsi utiliser une composition comprenant 50 g de matière active et 950 g de talc ; on peut aussi utiliser une composition comprenant 20 g de matière active, 10 g de silice finement divisée et 970 g de talc ; on mélange et broie ces constituants et on applique le mélange par poudrage.

L'invention concerne de plus un procédé de traitement des végétaux contre les champignons phytopathogènes.

Ce procédé est caractérisé en ce qu'il consiste à appliquer sur ces végétaux une quantité efficace d'une composition contenant comme matière active un composé selon la formule (II). Par "quantité efficace" on entend une quantité suffisante pour permettre le contrôle et la destruction des champignons présents sur ces végétaux. Les doses d'utilisation peuvent toutefois varier dans de larges limites selon le champignon à combattre, le type de culture, les conditions climatiques, et selon le composé utilisé.

En pratique des doses allant de 5 g/hl à 100 g/hl correspondant sensiblement à des doses de matière active par hectare de 50 g/ha à 1000 g/ha environ donnent généralement de bons résultats.

## REVENDICATIONS

1) Dérivé du cyano-2 benzimidazole caractérisé en ce qu'il répond à la formule générale :

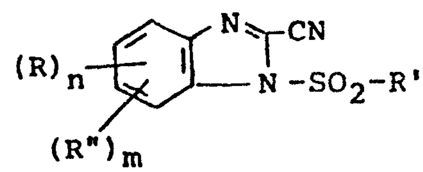

dans laquelle :

n représente un nombre entier égal à 0, 1 ou 2,

m représente un nombre entier égal à 1 ou 2,

R représente un atome d'halogène ou un radical alkyle inférieur, éventuellement substitué par un ou plusieurs atomes d'halogènes ; alkoxy inférieur éventuellement substitué par un ou plusieurs atomes d'halogènes ; alkylthio inférieur éventuellement substitué par un ou plusieurs atomes d'halogènes ; nitro ; cyano, étant entendu que, lorsque n est égal à 2, les substituants R peuvent être soit identiques soit différents ;

R' représente un radical alkyle ou cycloalkyle inférieurs, éventuellement substitués par un ou plusieurs atomes d'halogènes ; ou un radical amino éventuellement substitué par un ou deux radicaux alkyles inférieurs, identiques ou différents, eux-mêmes éventuellement substitués ; ou un atome d'azote substitué par deux radicaux formant avec cet atome d'azote un hétérocycle, lui-même éventuellement substitué, comportant de 4 à 6 chaînons et de 1 à 3 hétéroatomes dans le cycle ;

R" représente un radical alcénylthio inférieur, alcényloxy inférieur, alcynylthio inférieur,

alcynyloxy inférieur, aralkylthio, lesdits radicaux étant eux-mêmes éventuellement substitués, étant entendu que lorsque m est égal à 2, les substituants R"peuvent être soit identiques, soit différents, et que, dans ce qui précède, l'adjectif "inférieur", appliqué à un radical organique signifie que ce radical comporte au plus 6 atomes de carbone.

2) Composé selon la revendication 1 caractérisé en ce que :

n est égal à 0 ou à 1,

R représente un atome d'halogène ou le radical cyano, nitro, trifluorométhyle, trifluorométhoxy ou trifluorométhylthio,

R' représente le radical diméthylamino ou un radical alkyle ($C_1$-$C_3$) éventuellement halogéné,

m est égal à 1,

R" représente le radical propargylthio ou un radical benzylthio.

3) Composition pesticide, à usage agricole, utilisable notamment pour la lutte contre les champignons phytopathogènes, caractérisée en ce qu'elle comprend comme matière active un composé selon l'une des revendications 1 ou 2.

4) Composition selon la revendication 3 caractérisée en ce que, en plus de la matière active, elle comprend un support inerte et/ou un agent tensioactif utilisables en agriculture.

5) Composition selon la revendication 4 caractérisée en ce qu'elle comprend de 0,001 % à 95 % en poids de matière active et de 0 à 20 % en poids d'agent tensioactif.

6) Procédé de préparation d'un composé selon la revendication 1) caractérisé en ce qu'il consiste à faire réagir un cyano-2 benzimidazole de formule :

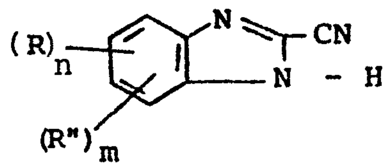

dans laquelle R, R", m et n ont la même signification que dans la revendication 1), ou un sel de métal alcalin ou d'ammonium de ce cyano-2 benzimidazole, sur un halogénure de formule :

$$X-SO_2R'$$

dans laquelle R' a la même signification que dans la revendication 1) et X représente un atome d'halogène.

7) Procédé selon la revendication 6) caractérisé en ce que la réaction entre le cyano-2 benzimidazole et l'halogénure s'effectue dans un solvant aprotique polaire, en présence d'un accepteur d'acide.

8) Procédé selon la revendication 6) caractérisé en ce que la réaction entre le sel de métal alcalin ou d'ammonium du cyano-2 benzimidazole et l'halogénure s'effectue dans un solvant aprotique polaire.

9) Procédé de traitement des végétaux contre les champignons phytopathogènes caractérisé en ce qu'il consiste à appliquer sur ces végétaux une quantité efficace d'un composé selon l'une des revendications 1 ou 2.

10)En tant que moyen spécialement conçu pour la mise en oeuvre du procédé selon la revendication 6), composé répondant à l'une ou l'autre des formules (III), (V) et (VI) ci-après :

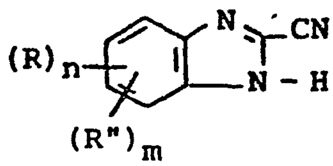

(III)

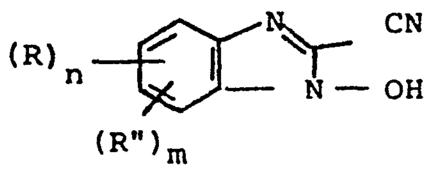

(V)

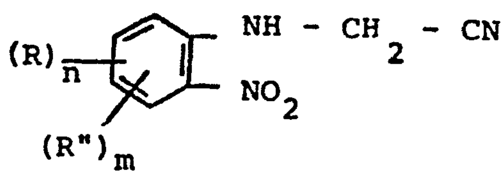

(VI)

dans lesquelles R, R", n et m ont la même signification que dans la revendication 1).

Office européen des brevets

**RAPPORT DE RECHERCHE EUROPEENNE**

**0181826**

Numero de la demande

EP 85 42 0193

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| A | EP-A-0 087 375 (RHONE-POULENC AGROCHEMIE)<br>--- | | C 07 D 235/24<br>A 61 K 31/415 |
| P,A | EP-A-0 152 360 (RHONE-POULENC AGROCHEMIE)<br>----- | | |
| | | | DOMAINES TECHNIQUES RECHERCHES (Int. Cl.4) |
| | | | C 07 D 235/00<br>A 61 K 31/00 |

Le présent rapport de recherche a été établi pour toutes les revendications

| Lieu de la recherche | Date d'achèvement de la recherche | Examinateur |
|---|---|---|
| LA HAYE | 20-12-1985 | DE BUYSER I.A.F. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant